(19) Europäisches Patentamt
European Patent Office
Office européen des brevets

(11) Numéro de publication: **0 088 667**
**B1**

(12) **FASCICULE DE BREVET EUROPÉEN**

(45) Date de publication du fascicule du brevet: **08.04.87**

(51) Int. Cl.[4]: **C 07 C 87/60**, C 08 G 59/50, C 08 G 18/38, C 08 G 18/32

(21) Numéro de dépôt: **83400381.6**

(22) Date de dépôt: **25.02.83**

(54) **Diamines aromatiques halogénées, leur procédé de fabrication, et leur application à la fabrication des polyuréthannes.**

(30) Priorité: **05.03.82 FR 8203666**

(43) Date de publication de la demande: **14.09.83 Bulletin 83/37**

(45) Mention de la délivrance du brevet: **08.04.87 Bulletin 87/15**

(84) Etats contractants désignés: **BE CH DE FR GB IT LI NL SE**

(56) Documents cité:
**DE-A-1 964 173**
**DE-A-2 339 237**
**FR-A-2 054 814**
**FR-A-2 382 430**

(73) Titulaire: **ATOCHEM, 4 & 8, Cours Michelet La Défense 10, F-92800 Puteaux (FR)**

(72) Inventeur: **Kervennal, Jacques, 134, rue Docteur Locard, F-69005 Lyon (FR)**
Inventeur: **Commandeur, Raymond, "Le Rocher" Avenue de Venaria, F-38220 Vizille (FR)**
Inventeur: **Huet, Jean- Marie, 16, rue du Petit Mont, F-27430 Saint- Etienne du Vauvray (FR)**

(74) Mandataire: **Rochet, Michel, ATOCHEM Département Propriété Industrielle, F-92091 Paris la Défense Cédex 42 (FR)**

**EP 0 088 667 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

L'invention concerne de nouveaux mélanges d'isomères de diamines aromatiques halogénées ayant pour squelettes hydrocarbonés des structures méthyldiphénylméthane ou polyméthyldiphénylméthane substituées par un ou plusieurs atomes d'halogènes. Ces mélanges d'isomères sont utilisables comme agents d'extension de chaîne dans la synthèse de polyuréthannes, ou comme agents de réticulation de résines époxydes, ou comme intermédiaires de synthèse en chimie organique, par exemple dans le domaine phytosanitaire.

Ces types de mélanges d'isomères asymétriques sont recherchés, mais n'ont jamais pu être obtenus qu'en mélanges avec leurs isomères symétriques.

C'est ainsi que des mélanges d'isomères voisins sont décrits dans le DE-A 2 339 237 s'agissant d'isomères asymétriques de diaminoéthyldiphénylméthane halogéné desquels on ne peut séparer les composés symétriques des composés asymétriques formés en cours de synthése.

La synthèse de ces mélanges d'isomères selon l'invention fait appel à deux étapes réactionnelles à partir d'un mélange d'isomères aromatiques halogénés: l'une de nitration, l'autre d'hydrogénation. L'action d'acide nitrique concentré en présence d'acide sulfurique peut conduire principalement à des isomères de dinitration dans lesquels chaque noyau aromatique porte une fonction nitrée.

Les fonctions nitrées sont ensuite hydrogénées en amines correspondantes.

Les structures hydrocarbonées, sont préparées par réaction de condensation de FRIEDEL et CRAFTS en présence de trichlorure d'aluminium ou de trichlorure ferrique, d'un chlorure de benzyle halogéné éventuellement substitué par un groupement méthyle, avec une molécule de toluène éventuellement halogénée ou de xylène halogénée les différents isomères aromatiques halogénés ainsi obtenus répondent à la formule générale suivante:

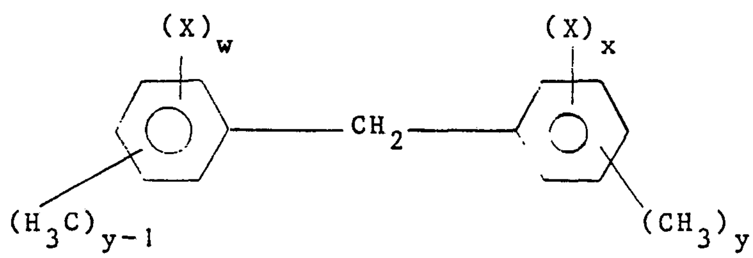

où X représente un atome de chlore ou de brome avec:
w = 1 ou 2
x = 0 à 2
y = 1 ou 2

Ils se présentent sous forme de mélanges d'isomères qui sont séparés du mllieu de réaction de condensation par distillation.

Parmi les hydrocarbures aromatiques servant de base à l'invention, la synthèse des isomères de (chloro ou bromo-benzyle) toluène peut se faire de différentes façons: F.A. VINGIELLO et J.G. VAN OOT dans le J. Amer. Chem. Soc., 73, 5070, (1951) et J. BLACKWELL et W.J. HICKINBOTTOM dans le J. Chem. Soc., 1405 (1961) ont décrit l'obtention de ces produits par réduction des cétones ou des alcools correspondants, mais de telles méthodes nécessitent de partir de composés élaborés et coûteux. Le brevet françals 2 237 866 décrit la préparation avec un rendement de 46 % de méthyl-2 chloro-4' diphénylméthane par condensation du chlorure d'orthométhylbenzyle sur le chlorobenzène en présence d'un catalyseur complexe de fluorure de bore et d'acide phosphorique; cette réaction peut être étendue au mélange des isomères du xylène, cependant, la monochloration des xylènes est assez peu sélective, car le deuxième méthyle a tendance à se chlorer également; de plus, dans l'étape de condensation FRIEDEL et CRAFTS, le chlorobenzène est désactivé et la cinétique de réaction est lente. Il est cependent préférable de faire réagir le chlorure de chloro-benzyle, obtenu par chloration photochimique ménagée de monochlorotoluène sur le coluène en présence de catalyseurs utilisés dans de telles réactions, par exemple A. TAKEMATSU, K. SUGITA, R. NAKANE dans le Bull. Chem. Soc. Japan, 51, 2082 (1978) ont essayé le trifluorure ds bore hydraté. L'intérêt de ce procédé réside dans le fait qu'on utilise le toluène comme seule matière première hydrocarbonée; on obtient ainsi un mélange de six isomères correspondant à la formule recherchée. Il se forme à côté, 10 à 15 % de produits de condensation à trois noyaux qu'on élimide lors de la distillation. Le mélange dôs isomères de (chloro ou bromo-benzyle) toluène ainsi préparés est ensuite dinitré.

La synthèse d'autres hydrocarbures aromatiquss de structure (polychloro)-méthyldiphénylméthane ou (polychloro)(polyméthyl)diphénylméthane est connue et décrite dans le brevet français 2 432 199 et le certificat d'addition 2 449 954; la chloration photochimique ménagée des chloroxylènes ou chlorotoluènes conduit aux chlorures de benzyle chlorés sur le noyau et substitués ou non par un méthyle qui peuvent se condenser sur des molécules de chlorotoluènes ou chloroxylènes en excès en présence de chlorure d'aluminium ou de chlorure ferrique. Les produits désirés sont séparés par distillation du milieu réactionnel et récupérés sous forme de melanges d'isomères. Ces réactions de condensation peuvent se faire entre 20°C et la température

de reflux des mélanges; un excès important de chlorotoluènes ou chloroxylènes favorise l'obtention de produits à deux noyaux.

L'étape de dinitration est effectuée de façon connue au moyen dun mélange d'acide nitrique et d'acide sulfurique concentrés à au moins 85 % et de préférence au moins 95 %. L'acide nitrique peut être placé en quantités stoechiométriques par rapport au composé aromatique, on utilise ainsi deux moles d'acide par mole de diphénylméthane substitué. Il est cependant avantageux d'opérer en présence d'un excès d'acide nitrique pouvant aller jusqu'à 20 % par rapport à la stoechiométrie. L'acide sulfurique peut etre utilisé en quantité équimolaire par rapport à l'acide nitrique, mais on peut le placer en excès ou en défaut. La réaction de nitration s'effectue entre 0° et la température d'ébullition des melanges, habituellement entre 0° et 50°C, le composé aromatique éant de préférence solubilisé dans un solvant tel que le chlorure de méthylène. La phase organique supérieure, est ensuite séparée des acides, neutralisée et évaporée à sec. Il est recommandé de travailler dans un réacteur muni de moyens d'aqitation et de régulation de température et d'introduction de gaz inertes.

Le mélange brut obtenu peut être hydrogéné tel quel. Il peut aussi être purifié, s'il contient une petite proportion de produits de coupure oxydante ou de mononitration. Pour ce faire on peut, par exemple, le dissoudre dans la quantité minimum de chlorure de méthylène, puis précipiter en ajoutant du méthanol ou de l'éther, un produit solide qui ne contient plus que des isomères de dinitration.

La deuxième étape constitue l'hydrogénation des dérivés nitrés en amines correspondantes. Cette étape s'effectue habituellement dans un réacteur résistant à la pression et possédant les moyens de contrôle et régulation classiques. L'hydrogénation peut être chimique, mais on préfère opérer sous pression d'hydrogèns, en présence d'un catalyssur à base de nickel, palladium, platine, ruthénium et autres. Dans ce cas, on utilise un réacteur d'hydrogénation permettant de travailler sous des pressions pouvant atteindre 100 bars. La réaction peut s'effectuer sans solvent à une température où le dérivé nitré est fondu, ou dans des solvents classiques d'hydrogénation tels que les alcools, le dioxanne, les éthers de l'éthylène glycol et autres. On utilise préférentiellement un catalyseur constitué de palladium déposé sur support à des teneurs comprises entre 1 et 10 %, ce qui permet d'opérer à des températures comprises entre 30 et 100°C et des pressions de 20 à 50 bars. Le rapport molaire

$$\frac{\text{dérivé dinitré}}{\text{palladium}}$$

n'est pas

impérativement fixé, mais il est de préférence compris entre 200 et 3 000. Après réaction et filtration du catalyseur, le solvant éventuellement utilisé est évaporé et le mélange des isomères de diamines obtenues peut être utilisé tel quel ou distillé. Le mélange d'isomères de l'invention répond à la formule générale suivante:

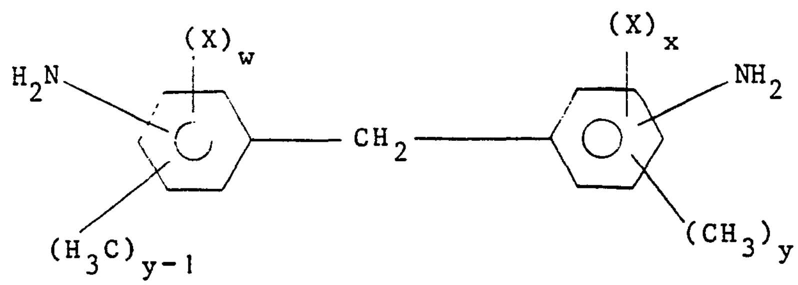

où X représente un atome de chlore ou de brome avec:

w = 1 ou 2

x = 0 à 2

y = 1 ou 2

Des diamines aromatiques halogénées et notamment le dichloro-3,3' diamino-4,4' diphénylméthane sont utilisées industriellement dans la fabrication d'élastomères polyuréthannes dans des procédés comportant deux étapes: on fait d'abord réagir un polyéther ayant un degré de fonctionnalité de 2 à 6 et une masse de 400 à 6 000 ou un polyester difonctionnel de masse 1 000 à 3 000 avec un diisocyanate en excès pour former un prépolymère liquide dans lequel des fonctions isocyanates sont bloquées dans des liaisons de type uréthanne.

On mélange ensuite à pression atmosphérique, le prépolymère avec l'amine fondue pour realiser l'extension de chaîne; la présence d'atomes d'halogènes sur les noyaux aromatiques diminue la réactivité de l'amine et permet d'empêcher la prise en masse immédiate du polymère. Les fonctions amines réagissent sur les groupements isocyanates libres pour former des urées et l'on a ainsi des enchaînements mixtes, de nature souple par les polyéthers, et, rigide par les fonctions uréthannes et urées. De tels procédés permettent d'obtenir des élastomères de structure contrôlée, mais ils exigent de travailler avec des amines à l'état fondu et il est donc intéressant de disposer de produits dont le point de fusion est le plus bas possible. Or, le point de fusion du dichloro-3,3' diamino-4,4' diphénylméthane est ainsi de 125°C, ce qui nécessite de le chauffer à cette température. Par contre, les nouveaux produits selon l'invention sont particulèrement intéressants, car ils se

présentent sous forme de mélanges d'isomères à point de fusion abaissé.

Les mélanges d'isomères de l'invention sont solubles dans des polyols courts et des polyols de masses plus élevées comprises entre 400 et 6 000. On peut ainsi atteindre des concentrations supérieures à 15 % en poids, ce qui permet de produire des élastomères d'uréthannes en une seule étape en faisant réagir directement ces solutions avec des diisocyanates.

Ces mélanges d'isomères peuvent être également utilisées comme agents de réticulation des résines époxy comme par exemple du diglycidyl éther du bisphénol A.

Les analyses sur les dérivés dinitrés et les mélanges d'isoméres sont effectuées par chromatographie en phase gazeuse. Dans le cas des produits de nitration, est utilisée une colonne en inox d'1/8", longue de 1,5 mètre, emplie d'un support chromosorb W. 80-100 mesh H.P. commercialisé par Johns Manville Prod. imprégné à 2 % de phase silicone XE-60 commercialisé par Applied Science Laboratories Inc.; lors de l'injection, la colonne est à 100° C et au bout de 5 minutes est portée à 220° C.

Dans le cas des amines, est utilisée une colonne en verre de 2 mètres, emplie de support chromosorb W.N.A.W. 60-80 mesh commercialisé par Johns Manville, imprégné à 5 % de KOH et 5 % d'Apiézon N commercialisé par Apiezon products Limited, en opérant en isotherme à 220° C.

Les identifications et déterminations de structure sont faites par couplage avec la spectrométrie de masse et, sur les produits purifiés, par résonance magnétique nucléaire du proton et du $^{13}C$.

**EXEMPLE 1**

On dissout 22 g (0,1 mole) des isomères de (chlorobenzyl) toluène dans 40 ml de chlorure de méthylène; en maintenant la température autour de 10° C sous agitation, on verse en une demi-heure un mélange de 15,5 g d'acide nitrique à 98,7 % et de 20 ml d'acide sulfurique à 96 %. Dès la fin de l'addition, on porte à reflux pendant 3 h 30 en maintenant l'agitation, puis refroidit. Deux phases se séparent, l'une inferieure acide, l'autre supérieure organique. On extrait la phase organique et lave deux fois les acides avec du chlorure de méthylène. On réunit les différentes fractions organiques, neutralise sur carbonate de potassium et evapore le solvant. On récupère ainsi 30,5 g d'un produit jaune clair visqueux dont l'analyse par chromatographie gazeuse couplée avec la spectrométrie de masse met en évidence les isomères de (nitro-chlorobenzyle)-nitrotoluène.

On place 20 g de ce mélange d'isomères dans l'autoclave précedemment décrit et ajoute 200 ml de méthanol ainsi que 0,2 g de catalyseur constitué de palladium déposé sur charbon à 5 %. Après avoir balayé le réacteur avec de l'azote, on y introduit 40 bars d'hydrogène et le chauffe, sous agitation, jusqu'à 60° C. Après 3 h 20 de réaction, on laisse refroidir, décomprime et récupère le mélange réactionnel qu'on filtre pour éliminer le catalyseur. Le méthanol est évaporé à sec et on obtient 16 g d'un produit brun-foncé très épais. L'analyse par couplage de la chromatographie gazeuse avec la spectrométrie de masse montre qu'il s'agit des isomères d'(amino-chlorobenzyle)-aminotoluène correspondant à la formule générale donnés dans le descriptif, dans laquelle $X = Cl$, $w = 1$, $x = 0$, $y = 1$ et $z = 0$.

**EXEMPLE 2**

On dissout 138 g du mélange d'isomères de (chlorobenzyl) toluènes dans 240 ml de chlorure de méthylèns. En opérant de la même façon qua dans l'exemple 1, on additionne un mélange de 93 g d'acide nitrique à 98,3 % et de 120 ml d'acide sulfurique à 96 % On chauffe à reflux pendant 4 h 30 puis refroidit et extrait la phase organique. On évapore le solvant jusqu'à la limite de solubilité des isomères de dinitration puis on ajoute du methanol et précipite ainsi un solide blanc qu'on filtre et lave au méthanol. On récupère après séchage 21,5 g de solide blanc de point de fusion 183° C. L'analyse par chromatographie gazeuse met en évidence la présence nettement majoritaire d'un isomère de dinitro(chlorobenzyl) toluène que l'étude en résonance magnétique nucléaire identifie comme étant le dinitro-3,3' méthyl-4 chloro-4' diphénylméthane:

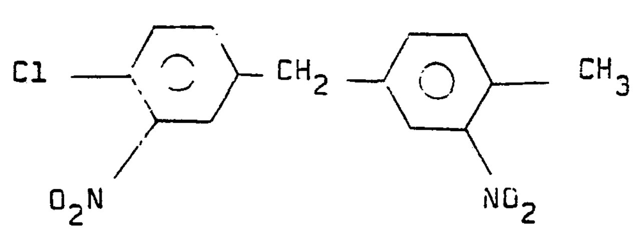

Le filtrat méthanolique contient le reste de cet isomère majoritaire ainsi que les autres isomères de dinitration.

20 g du précipité sont hydrogénés suivant le mode opératoire décrit dans l'exemple 2 à l'aide de 200 ml de méthanol et 0,4 g decatalyseur Pd sur charbon à 5 %. On récupère, après évaporation du méthanol, 16 g de

produit brun-foncé, pâteux, constitué essentiellement de diamino-3,3' méthyl-4 chloro-4' diphénylméthane.

**EXEMPLE 3**

Dans un réacteur aqité de 250 ml, on place, sous atmosphère d'azote, 25,1 g (0,1 mole) d'un mélange d'isomères de (chlorobenzyl)-chlorotoluène synthétisés à partir de chlorotoluènes selon la description faite dans le brevet français n° 2 432 199. On ajoute 40 ml de chlorure de méthylène et introduit en une demi-heure entre 5 et 15°C un mélange de 13,5 g d'acide nitrique à 98,3 % et 20 ml d'acide sulfurique à 96 %. On chauffe ensuite à 42°C pendant 3 h 30 puis refroidit et laisse décanter. On récupère la phase organique supérieure et lave par du chlorure de méthylène la phase acide inférieure. Les extraits organiques de soutirage et de lavage sont mélangés, neutralisés avec du carbonate de potassium et séchés à l'aide de sulfate de sodium. On obtient une solution limpide de couleur orangée qu'on évapore à sec. On recueille ainsi 32 g de liquide orangé très visqueux correspondant au mélange d'isomères de (nitro-chlorobenzyl)-nitro-chlorotoluène.

On hydrogène un échantillon de 15 g de ce liquide, dans un autoclave en acider inox muni d'une agitation magnétique, en présence de 0,15 g de catalyseur palladium eur charbon à 5 % (ENGELHARD) et 150 ml de méthanol. En opérant à 35 bars et 50°C, la réaction dure 1 h 30, puis on décharge le réacteur et récupère après évaporation du solvant 13,5 g d'un solide orangé de point de fusion 45-47°C. L'analyse par chromatographie gazeuse montre qu'il s'agit des isomères de la diamine attendue, répondant à la formule générale des diamines de l'invention dans laquelle X = Cl, z = 0, w = x = y = 1.

**EXEMPLE 4**

On dissout 35 g du mélange liquide d'isomères de (nitrochlorobenzyl)-nitrochlorotoluène préparés d'une façon identique à la synthèse décrite dans l'exemple 3. On dissout ce liquide dans le minimum de chlorure de méthylène et ajoute de l'éther éthylique jusqu'à formation d'un précipité blanc floconneux qu'on recueille par filtration. Après lavage à l'éther et séchage, ce produit blanc de point de fusion: 158°C est analysé par résonance magnétique nucléaire. Il s'agit d'un mélange enrichi d'isomères dont le constituant majoritaire correspond à la formule suivante:

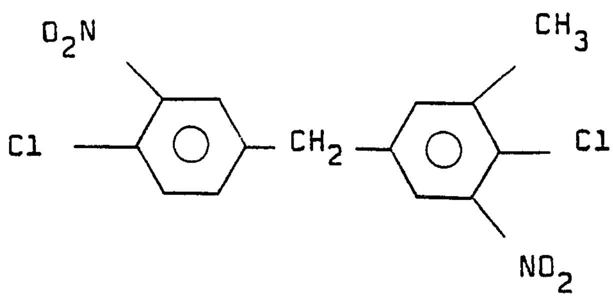

Ce précipité blanc est hydrogéné dans les conditions décrites dans l'exemple 1 pour conduire aux amines correspondantes.

**EXEMPLE 5**

27,9 g (0,1 mole) des isomères de (chloroparaméthylbenzyle)chloroparaxylène préparés par réaction de condensation de FRIEDEL et CRAFTS de chlorure de chloro-paraméthylbenzyle sur le chloroparaxylène, sont dissous dans 40 ml de chlorure de méthylène. On maintient la température autour de 10°C, agite et introduit en une demi-heure un mélange de 15,5 g d'acide nitrique à 98,7 % et de 20 ml d'acide sulfurique à 96 %. On porte à reflux pendent 3 h 30, puis refroidit. On récupère la phase organique supérieure et lave les acides avec du chlorure de méthylène. Les phases organiques sont rassemblées et traitées avec du carbonate de potassium, puis le solvant est évaporé. On récupère ainsi 36 g de produits de dinitration.

On hydrogène 20 g de ce mélange suivant le mode opératoire décrit dans l'exemple 1, avec 0,2 l de méthanol et 0,4 g de catalyseur Pd/charbon à 5 %. On récupère, après distillation du méthanol, 16,5 g d'un produit cristallisé rougeâtre de point de fusion 73°C, correspondant essentiellement aux isomères d'(amino-chloro-paraméthyl-benzyle)-amino-chloroparaxylène de formule générale:

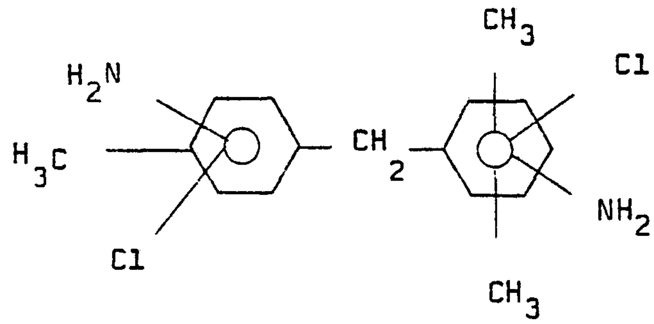

**EXEMPLE 6**

A 200 g de polyadipate d'éthylène glycol d'indice d'hydroxyle 56, préalablement déshydraté sous vide à 130°C, on ajoute, à 100°C, 29,2 g d'un mélange d'isomères de 80 % de toluène diisocyanate 2,4 et de 20 % de 2,6 (TDI). On maintient ce mélange sous agitation à 100°C pendant 1 h. On obtient de cette manière un prépolymère à fonctions isocyanates réactives. On introduit, sous agitation, 12,1 g d'un mélange d'isomères à l'état fondu d'(amino-chlorobenzyl)-aminochlorotoluène dont la préparation est décrite dans l'exemple 3. On met sous vide pendant 30 secondes. Le mélange de réaction liquide est ensuite coulé dans un moule préchauffé où il se solidifie en guelques minutes. Après 24 heures de chauffage à 100°C, on obtient un élastomère de polyuréthanne ayant d'excellentes propriétés.

**EXEMPLE 7**

A un polyéther triol propoxylé, puis éthoxylé sur une base triméthylolpropane ($I_{OH}$ = 35,

$$\frac{OE}{OE + OP} =$$

14 %; % OH primaire = 77), on ajoute le mélange d'isomères d'amines obtenue suivant l'exemple 3 à raison de 100 g de polyol pour 7,5 g d'amines. Ce mélange homogène est additionné, à température ambiante et sous agitation, à 17,8 g de T.D.I. On met sous vide 30 secondes et on coule le mélange réactionnel dans un moule qui est porté à 120°C pendant 12 heures. On obtient un élastomère polyuréthanne possédant de bonnes propriétés mécaniques.

**EXEMPLE 8**

On utilise une résine de diglycidyl éther du bisphénol A (DGEBA) commercialisée par la société CIDA-GEIGY sous la référence GY 250 possédant les caractéristiques suivantes: 5,3 fonctions époxy/kg; 0,5 éq Cl/kg; 0,5 fonction OH/kg. La réticulation est effectuée avec le mélange des isomères de diamines préparé à l'exemple 5 dans les conditions suivantes: mélange de 100 g de DGEBA additionné de 35,8 g d'amine portée à 80°C. On coule le mélange obtenu dans un moule de dimensions intérieures 12 x 1,5 x 1 cm permettant d'obtenir un barreau de résine. La réticulation nécessite un étuvage de 2 h à 100°C puis 2 h à 150°C. Après démoulage on obtient un barreau présentant de bonnes propriétés mécaniques.

**EXEMPLE 9**

On nitre, à l'aide de 18 g d'acide nitrique à 98,7 % et de 26,5 g d'acide sulfurique à 96 % dans les conditions décrites dans l'exemple 1, 30,6 g de mélange d'isomères de (dichlorobenzyle) - dichlorotoluène préparé par condensation de FRIEDEL et CRAFTS de chlorure de dichlorobenzyle sur le dichlorotoluène. On récupère 39 g d'un mélange liquide visqueux de couleur jaune-orangée dont on hydrogène 38,2 g en présence de 1,9 g de

catalyseur palladium sur charbon à 5 % (ENGELHARD) dans 200 ml de méthanol, à 60°C sous 40 bars. Après refroidissement, détente et filtration du catalyseur, on récupère 32 g d'un mélange de couleur rose des diamines correspondant à la formule brute du descriptif dans laquelle X = Cl, w = 2, x = 2, y = 1, z = 0 et de point de fusion 87°C.

**EXEMPLE 10**

On dissout 26,1 g du mélange d'isomères de (bromo-benzyl) toluène dans 40 ml de chlorure de méthylène. En opérant de la même façon que dans l'exemple 1, on additionne un mélange de 18 g d'acide nitrique à 98,7 % et de 26,5 g d'acide sulfurique à 96 % et on laisse à reflux pendant 3 h 30. Après décantation, extraction et neutralisation par $Na_2CO_3$, la distillation du chlorure de méthylène conduit à 35 g d'un produit marron clair pâteux correspondant au mélange d'isomères de (nitrobromobenzyl)-nitrotoluène.

On hydrogène 29 g de ce mélange, en présence de 0,29 g de catalyseur palladium sur charbon à 5 % et de 0,29 l de méthanol. On opère à 40 bars et 60°C, puis on décharge le réacteur et récupère, après évaporation du solvant, 24 g d'un solide marron foncé de point de fusion 96°C, correspondant aux isomères de diamine attendue, répondant à la formule générale des diamines de l'invention dans laquelle X = Br, w = 1, y = 1, x = z = 0.

**Revendications**

1 - Mélange d'isomères de diamines aromatiques halogénées de formule:

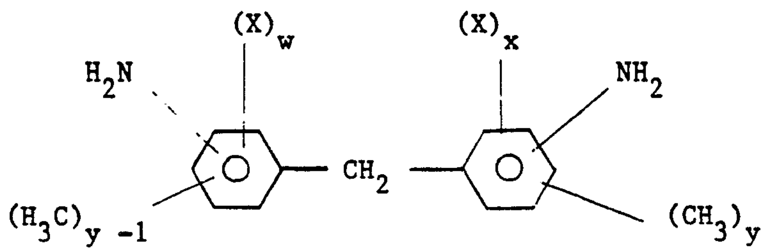

où X représente un atome de chlore ou de brome avec:
w = 1 ou 2
x = 0 à 2
y = 1 ou 2

2 - Diamines aromatiques halogénées selon la revendication 1 caractérisées en ce qu'il s'agit des mélanges d'isomères d'(aminochlorobenzyl)aminotoluène.

3 - Diamines aromatiques halogénées selon la revendication 1 caractérisées en ce qu'il s'agit des mélanges d'isomères d'(amino-chlorobenzyl)-aminochlorotoluène.

4 - Diamines aromatiques halogénées selon la revendication 1 caractérisées en ce qu'il s'agit des mélanges d'isomères d'(amino-chloro-paraméthylbenzyle)-amino-chloroparaxylène.

5 - Procédé de fabrication des mélanges d'isomères de diamines aromatiques halogénées selon les revendications 1 à 4 caractérisé en ce que les composés de la formule:

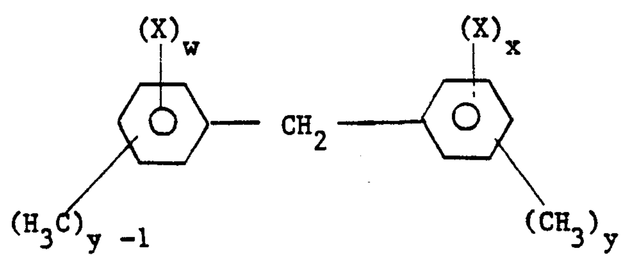

où X représente un atome de chlore ou de brome avec:
w = 1 ou 2
x = 0 à 2
y = 1 ou 2

sont successivement dinitrés puis hydrogénés.

6 - Procédé selon la revendication 5 caractérisé en ce que la nitration s'effectue au moyen d'un mélange d'acide nitrique et d'acide sulfurique de concentration supérieure à 85 %.

7 - Procédé selon l'un des revendications 5 à 6 caractérisé en ce que l'hydrogénation s'effectue en présence de palladium comme catalyseur.

8 - Application des mélanges d'isomères de diamines aromatiques halogénées selon l'une des revendications 1 à 7 comme agents d'extension de chaînes dans la synthèse des polyuréthannes.

9 - Application des mélanges d'isomères de diamines aromatiques halogénées selon l'une des revendications 1 à 7 comme agents de réticulation des résines époxy.

**Claims**

1. Mixture of isomers of halogenated aromatic diamines of formula:

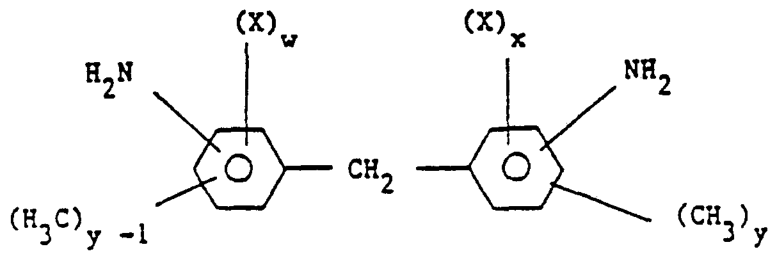

where X denotes a chlorine or bromine atom with:
w = 1 or 2
x = 0 to 2
y = 1 or 2

2. Halogenated aromatic diamines according to Claim 1, characterized in that they are mixtures of isomers of (aminochlorobenzyl)aminotoluene.

3. Halogenated aromatic diamines according to Claim 1, characterized in that they are mixtures of isomers of (aminochlorobenzyl)aminochlorotoluene.

4. Halogenated aromatic diamines according to Claim 1, characterized in that they are mixtures of isomers of (aminochloro-para-methylbenzyl)aminochloro-para-xylene.

5. Process for the manufacture of mixtures of isomers of halogenated aromatic diamines according to Claims 1 to 4, characterized in that the compounds of the formula:

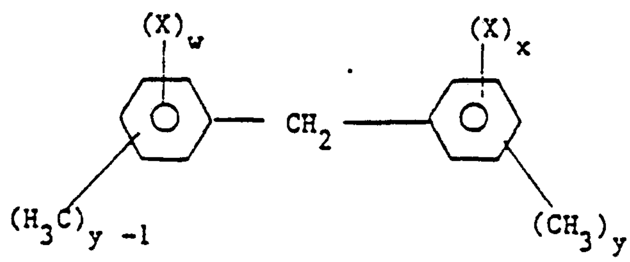

where x denotes a chlorine or bromine atom, with:
w = 1 or 2
x = 0 to 2
y = 1 or 2
are successively dinitrated and then hydrogenated.

6. Process according to Claim 5, characterized in that the nitration is carried out using a mixture of nitric acid and of sulphuric acid at a concentration above 85%.

7. Process according to either of Claims 5 and 6, characterized in that the hydrogenation is carried out in the presence of palladium as a catalyst.

8 Use of the mixtures of isomers of halogenated aromatic diamines according to one of Claims 1 to 7 as chain extenders in the synthesis of polyurethanes.

9. Use of the mixtures of isomers of halogenated aromatic diamines according to one of Claims 1 to 7 as crosslinking agents for epoxy resins.

**Patentansprüche**

1. Gemisch von Isomeren der halogenierten aromatischen Diamine mit folgender Formel

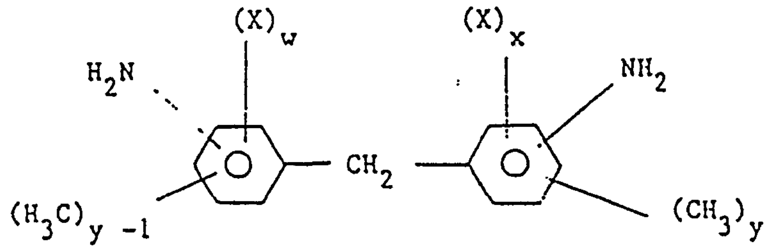

in der X ein Chlor- oder Bromatom und
w = 1 oder 2
x = 0 bis 2
y = 1 oder 2 ist.

2. Halogenierte aromatische Diamine nach Anspruch 1, dadurch gekennzeichnet, daß es sich um ein Gemisch der Isomeren des (Amino-chlorbenzyl)aminotoluols handelt.

3. Halogenierte aromatische Diamine nach Anspruch 1, dadurch gekennzeichnet, daß es sich um ein Gemisch der Isomeren des (Amino-chlorbenzyl)aminochlortoluols handelt.

4. Halogenierte aromatische Diamine nach Anspruch 1, dadurch gekennzeichnet, daß es sich um ein Gemisch der Isomeren des (Amino-chlor-paramethylbenzyl)aminochlorparaxylols handelt.

5. Verfahren zur Herstellung der Isomerengemische von halogenierten aromatischen Diaminen gemäß den Ansprüchen 1 bis 4, dadurch gekennzeichnet, daß Verbindungen der allgemeinen Formel

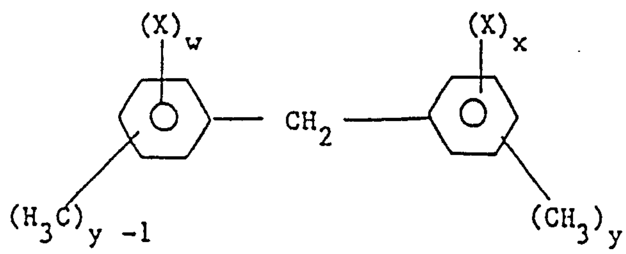

in der X ein Chlor- oder Bromatom und
w = 1 oder 2
x = 0 bis 2
y = 1 oder 2 ist,
zunächst dinitriert und dann mit Wasserstoff reduziert werden.

6. Verfahren nach Anspruch 5, dadurch gekennzeichnet, daß die Nitrierung mittels einer Mischung aus Salpetersäure und Schwefelsäure mit einer Konzentration von mindestens 85 % durchgeführt wird.

7. Verfahren nach den Ansprüchen 5 und 6, dadurch gekennzeichnet, daß die Reduktion mit Wasserstoff in Gegenwart von Palladium als Katalysator durchgeführt wird.

8. Verwendung der Isomerengemische der halogenierten aromatischen Diamine aus den Ansprüchen 1 bis 7 als Mittel zur Kettenverlängerung bei der Synthese von Polyurethanen.

9. Verwendung der Isomerengemische der halogenierten aromatischen Diamine aus den Ansprüchen 1 bis 7 als Mittel bei der Vernetzung von Epoxidharzen.